# EUROPEAN PATENT SPECIFICATION

(11) **EP 3 075 362 B1**
(45) Date of publication and mention of the grant of the patent: **31.01.2018**
(21) Application number: 16162180.0
(22) Date of filing: 24.03.2016
(51) Int. Cl.: A61F 5/445

(54) **OSTOMY BAG FOR EXTENDED WEAR**
OSTOMIEBEUTEL MIT LÄNGERER TRAGBARKEIT
POCHE DE STOMIE À PORT PROLONGÉ

(30) Priority: 24.03.2015 EP 15160563
(43) Date of publication of application: 05.10.2016
(73) Proprietor: OxMed International GmbH, 46446 Emmerich am Rhein (DE)
(72) Inventor: de Weert, Heleen, 7001 KJ Doetinchem (NL)
(74) Representative: Nederlandsch Octrooibureau

(56) References cited:
- WO-A2-2005/082271
- GB-A- 2 116 433
- GB-A- 2 259 255
- GB-A- 2 334 678
- US-A- 4 938 750
- US-A- 5 690 621

## Description

### Field of the invention

The present invention relates to an ostomy bag assembly having an outer bag and an inner, user replaceable bag, suitable for receiving bodily waste, e.g. in the form of a colostomy, ileostomy or urostomy solution.

### Prior art

British patent publication GB-A-22 59 255 discloses an ostomy bag having two vents, one of which is filtered and the other of which is unfiltered. Sealing tape is used to seal the vents and can be peeled back to open them. In use, the vent is opened so that by pulling the walls of the bag apart, air is drawn through the vents into the bag. The vent is then closed to trap air in the bag and keep the front wall away from the rear wall in the region of the opening, so that waste does not collect opposite the opening and obstruct it.

International patent application WO 2005/082271 discloses a disposable inner bag liner for an ostomy appliance, see the figures, wherein the inner bag liner is capable of forming a bag inside an outer receiving member.

European patent publication EP-A-0 768 849 discloses a drainage bag system for receiving bodily waste having a water-impermeable outer bag and a water-impermeable inner bag. The inner bag has a first opening, covered by a gas-permeable material, and the second bag has a second opening, covered by a flatus filter. Flatus gasses can thus escape from the inner bag to the outer bag, and hence via the flatus filter to the outside environment.

### Summary of the invention

The present invention seeks to provide an improved ostomy bag assembly allowing extended wear by a patient and improved gas accumulation control. The present invention relates to an ostomy bag assembly according to claim 1. The outer bag is provided with a three position filter device, wherein in a first position the filter device is arranged to guide gas out of the outer bag via a gas filter cartridge, in a second position the filter device is arranged to allow unfiltered gas to escape from the outer bag, in a third position the filter device is arranged to block gas from escaping from the outer bag, and wherein the three position filter device comprises a shutter member in movable engagement with a filter body attached to the outer bag.

The ostomy bag assembly of the present invention has the advantage that the three position filter device allows a user to conveniently control gas accumulation within the ostomy bag assembly. The filter device not only allows for gas discharge filtration through the gas filter cartridge, but the filter device also allows venting of the ostomy bag assembly to prevent over inflation as well as exhaust gas blocking for preventing the development of a vacuum within the ostomy bag assembly ('pancaking'). Changing between positions of the filter device is straightforward and requires very little effort from the user.

In a further embodiment, the gas filter cartridge is a replaceable gas filter cartridge, allowing easy replacement by the user when deemed necessary. The gas filter cartridge may comprises an active gas filtering ingredient, e.g. active carbon, and optionally a scent ingredient, e.g. a perfume, fragrance, masking odour, etc. which further enhances comfort of use of the ostomy bag assembly.

The first, second and third positions form a linear, e.g. a sliding, arrangement, or alternatively form a circular arrangement. In an embodiment the filter device is positioned on a top part of the outer bag, and furthermore e.g. on the same side as the outer bag main aperture, which enhances wearability and accessibility for a user. Other locations of the filter device on the outer bag are also conceivable.

In a further embodiment, a coupling between the inner bag and the outer bag is provided by a sealing arrangement, the inner bag comprising an inner bag coupling part surrounding the inner bag main aperture, and the outer bag comprising an outer bag coupling part surrounding the outer bag main aperture, the sealing arrangement comprising an O-ring seal and a secondary seal, wherein the secondary seal comprises an adhesive tape attached to respective mating surfaces on the inner bag coupling part and the outer bag coupling part. The adhesive tape is e.g. of an easy-to-remove type, and the secondary seal may be provided with a release paper, which is removable directly before use. In addition, the release paper may be provided with a tear off tab, and/or comprises two half circular parts. This coupling is very user-friendly and easy to use, while at the same time ensuring sufficient sealing between the various components of the ostomy bag assembly.

In a further embodiment, an inner edge surface of the outer bag coupling part and an inner edge surface of the inner bag coupling part form a continuous surface. This allows easy wipe and clean actions by the user, as there is no ridge where accumulation may occur.

The outer bag coupling part comprises a plurality of outer bag coupling posts and the inner bag coupling part comprises a plurality of alignment apertures in a further embodiment. The plurality of outer bag coupling posts and the plurality of alignment apertures are configured for aligning the inner bag and outer bag coupling part, and thus the outer and inner bag coupling part, allowing easy attachment of the inner bag. In an even further embodiment, the one or more outer bag coupling posts, having a first section with a diameter larger than the inner diameter of an inner bag coupling location aperture, and a second section with a diameter smaller than the inner diameter of an inner bag coupling location aperture. Such a snap fit construction will make a reassuring click for the user, and the free float over the second section will allow easy removal of release paper.

### Short description of drawings

The present invention will be discussed in more detail below, using a number of exemplary embodiments, with reference to the attached drawings, in which
Figure 1a shows a cross sectional view of an embodiment of an ostomy bag assembly according to the present invention;
Figure 1b shows the embodiment of figure 1a, wherein the inner and outer bag are coupled;
Figure 2a shows a partial cross sectional view of an embodiment of the inner and outer bag coupling parts;
Figure 2b shows a partial cross sectional view of figure 2a, where the inner and outer bag coupling parts are coupled to each other;
Figure 3 shows a partial view of an inner bag according to an embodiment of the present invention, directly prior to use;
Figure 4 shows an embodiment of a three position filter device according to the present invention;
Figure 5a shows an embodiment of a three position filter device in a first position according to the present invention;
Figure 5b shows an embodiment of a three position filter device in a second position according to the present invention;
Figure 5c shows an embodiment of a three position filter device in a third position according to the present invention;
Figure 6 shows a side view, partially in cross section, of a further embodiment of the present invention ostomy bag assembly;
Figure 7a and 7b show three dimensional views of an upper and lower part of the ostomy bag assembly of Figure 6.

### Detailed description of exemplary embodiments

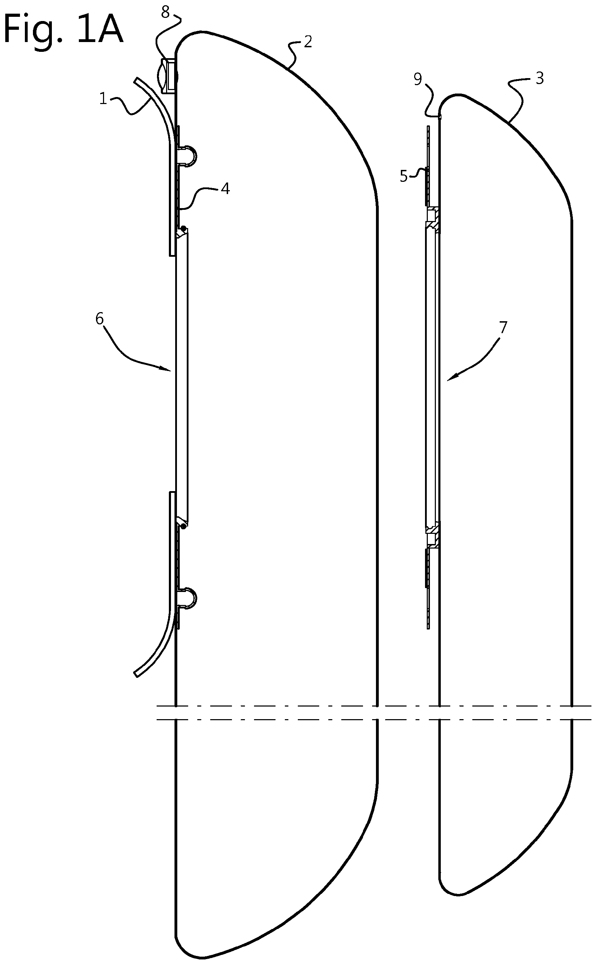

Figure 1a shows an embodiment of an ostomy bag assembly according to the present invention. In the embodiment shown the ostomy bag assembly comprises an outer bag 2 and an inner bag 3 that serves to store bodily discharge such as bowel matter. The outer bag 2 is larger than the inner bag 3 and worn on a patient via, for example, a hydrocolloid base plate coupling 1 attached to the skin when in use. The base plate coupling 1 is attached to an edge of an outer bag main aperture 6 arranged to extend around a stoma outlet. In many embodiments the outer bag 2 can be opened longitudinally on the outer bag 2 via a centrally positioned grip sealing member arranged thereon (not shown).

The inner bag 3 comprises an inner bag main aperture 7 which in use is aligned with the outer bag main aperture 6 of the outer bag 2, and is further provided with a liquid sealing and gas permeable barrier device 9 to allow gas to be discharged from the inner bag 3. The outer bag 2 is provided with a three position filter device 8 to allow gas accumulating in the outer bag 2 to be filtered and/or discharged to the outside environment. In general terms, the three position filter device 8 is arranged to have a first, second and third operational position each allowing a different amount of gas to be guided out from the outer bag 2. For this, the three position filter device 8 comprises a shutter member 27 in movable engagement with a filter body 25 attached to the outer bag 2, as explained in further detail with reference to the exemplary embodiments described below.

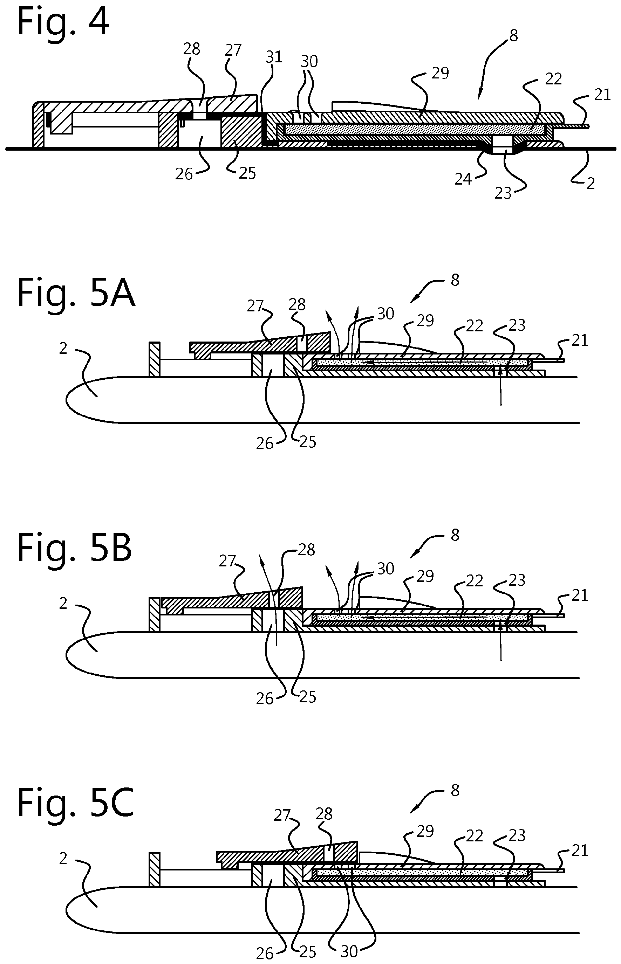

In one group of embodiments, in a first position the filter device 8 is arranged to guide gas out of the outer bag 2 via a gas filter cartridge 21 (see description of figure 4 and 5a-c below for further details), in a second position the filter device 8 is arranged to allow unfiltered gas to escape from the outer bag 2, and in a third position the filter device 8 is arranged to block gas from escaping from the outer bag 2.

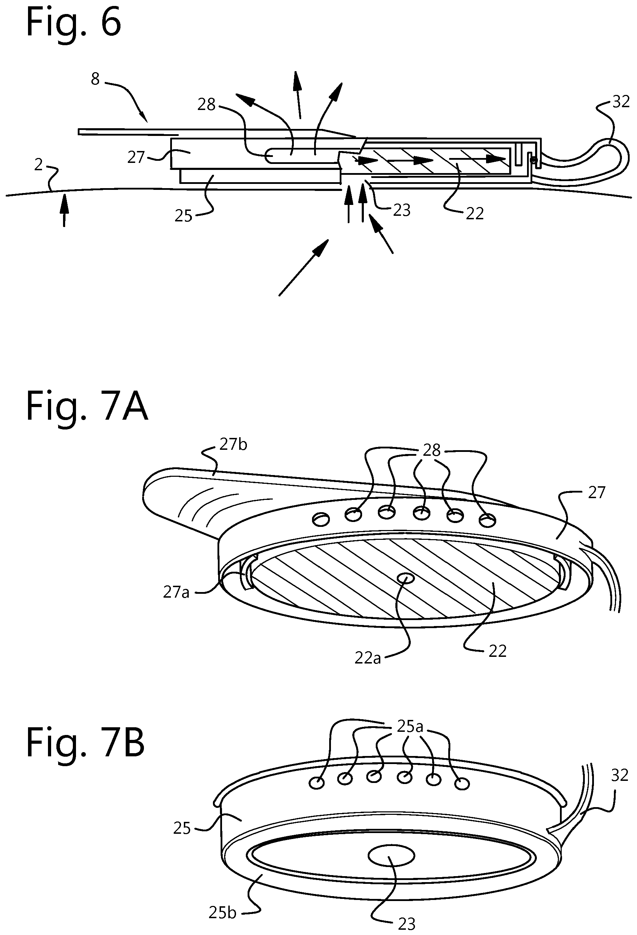

In a further group of embodiments, in a first position the filter device 8 is arranged to guide gas out of the outer bag 2 via a gas filter cartridge 21, in a second position the filter device 8 is arranged to guide gas out of the outer bag 2 via a gas filter cartridge 21 with a constricted flow, and in a third position the filter device 8 is arranged to block gas from escaping from the outer bag 2. In the second position, e.g. only 50% of the flow possible in the first position is allowed to escape from the outer bag 2, see the description of figure 6 and 7a-b below.

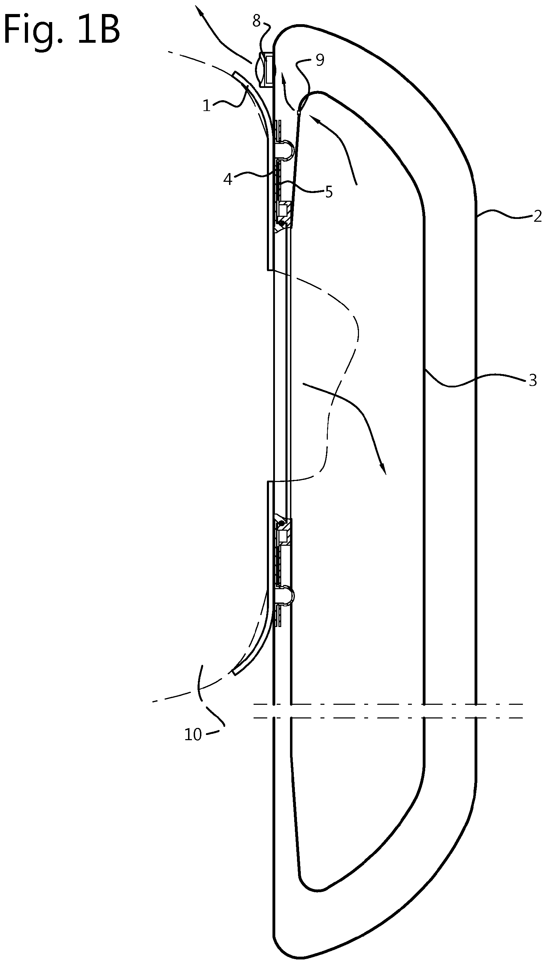

Figure 1b depicts the embodiment of the ostomy bag assembly as shown in figure 1a, when the outer bag 2 and the inner bag 3 are coupled together. The depicted arrangement of the outer bag 2 and inner bag 3 corresponds to the ostomy bag assembly being in use. As shown, the inner bag 3 is fully enclosed by the outer bag 2 so that the outer bag 2 prevents damage of the inner bag 3 whilst offering a safe and hygienic storage and possible replacement thereof. The outer bag 2 is constructed of a multilayer film, one of which layers provides a barrier that prevents odour form being transmitted through the film, e.g. using an oxygen barrier film. The only exit of smell carrying molecules in the ostomy bag assembly of the present invention embodiments is then via the (carbon) filter device 8 which treats the gas prior to exiting.

In a typical embodiment the outer bag 2 comprises an outer bag coupling part 4 and the inner bag 3 comprises an inner bag coupling part 5. The outer and inner coupling parts 4, 5 are in mutual sealing engagement when the inner bag 3 is properly fitted within the outer bag 2 during use, so that any discharge or spilling from the inner bag 3 to the outer bag 2 is prevented.

In an advantageous embodiment, the filter device 8 is positioned on a top part of the outer bag 2, as shown in the embodiment of figure 1a and 1b. As stoma discharge matter tends to accumulate in a bottom region of the inner bag 3, positioning the filter device 8 in a top part of the outer bag 2 prevents blocking of the filter device 8 should discharge matter be able to enter the outer bag 2, which would then accumulate in a bottom region thereof.

In another advantageous embodiment, the filter device 8 is positioned on the outer bag 2 on the same side as the outer bag main aperture 6, again as shown in the embodiment of figure 1a and 1b. This embodiment provides a filter device 8 that is out of sight and hidden behind the outer bag 2, which is generally perceived as offering an improved visual appeal. Also the outer bag 2 provides a cushioning effect in use to limit the relative hardness of the housing of the filter device 8 (see detailed description with reference to figure 4 below) against the patient's skin.

Other locations of the filter device 8 on the outer bag 2 are conceivable, e.g. in other applications where it may be advantageous that the filter device 8 is on the exposed side of the outer bag 2 during use.

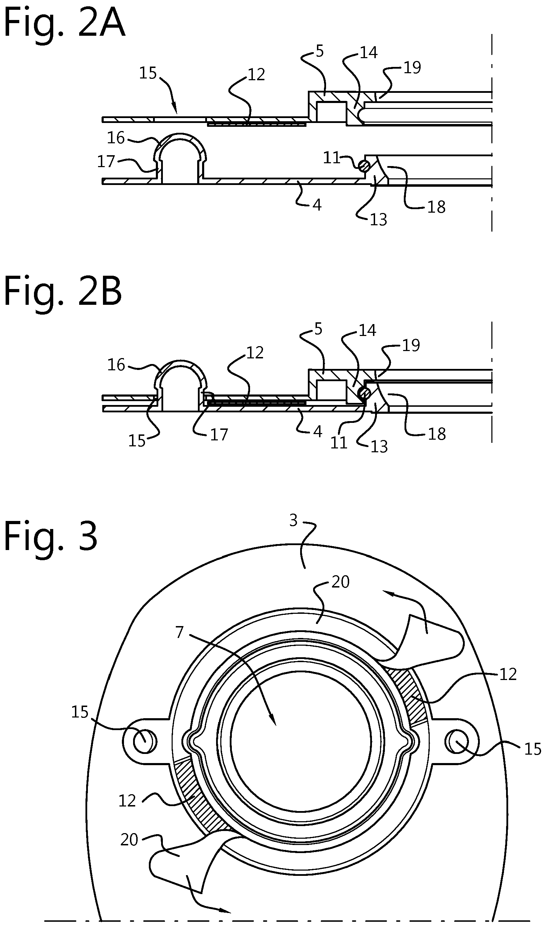

Figure 2a and 2b each show a partial cross sectional view of an embodiment of an inner bag coupling part 5 and an outer bag coupling part 6 according to the present invention. Figure 2a depicts a first configuration wherein the inner bag 3 and outer bag 2 are separate, and figure 2b depicts a second configuration wherein the inner 3 and outer bag 2 are firmly fitted together.

In the advantageous embodiments shown, the ostomy bag assembly comprises a coupling between the inner bag 2 and the outer bag 3 provided by a sealing arrangement. The inner bag 3 comprises an inner coupling part 5 surrounding the inner bag main aperture 7 and the outer bag 2 comprises an outer bag coupling part 4 surrounding the outer bag main aperture 6. The sealing arrangement in the embodiment as shown in Fig, 2a and 2b comprises an O-ring seal 11 and a secondary seal 12. The O-ring seal 11 is e.g. provided in a circumferential recess of an outwardly extending ring 13 which is part of the inner bag coupling part 4. In use, it fits into a circumferential recess in an inwardly extending ring 14 of the inner bag coupling part 5. The secondary seal 12 comprises an adhesive tape attached to respective mating surfaces on the inner bag coupling part 5 and the outer bag coupling part 4. The mating surfaces may be implemented as circumferential surface parts of the inner and outer coupling parts 5, 4.

In an embodiment the adhesive tape is fixedly attached to the inner bag coupling part 5, ensuring optimal adhesion between the inner and outer bag coupling part 5, 4 and preventing spontaneous release thereof as well as providing further sealing there between.

According to the embodiment shown in figure 2b, the sealing arrangement allows for a secure and tight connection between the inner and outer coupling part 5, 4 of the ostomy bag assembly. The inner and outer bag coupling part 5, 4 may be interlocked through e.g. a snap or press fit, whereby the O-ring 11 is sufficiently compressed between the inner and outer bag coupling part 5, 4 for providing a gas tight seal, which also provides a seal for liquids, solids and semi-solids. Once fitted together, the adhesive tape of the secondary seal 12 then provides additional adhesive engagement and sealing between the inner and outer bag coupling parts 5, 4 and the interlocking arrangement is prevented from spontaneous release.

Since the inner bag 3 requires replacement at particular time intervals, the interlocking arrangement of the inner and outer bag coupling part 5, 4 in the mutual positioning as shown in figure 2b must be easy to release by a user or patient.

In an advantageous embodiment, making the interlocking arrangement between the inner and outer bag coupling part 14, 13 releasable may be achieved by having the adhesive tape implemented as an easy to remove type, e.g. by providing a releasable but sticky surface on the side facing away from the inner bag coupling part 5. This embodiment provides sufficient additional adhesive engagement between the inner and outer bag coupling part 5, 4 for preventing spontaneous release thereof and ensuring additional sealing, whilst retaining a releasable interlocking arrangement between the inner and outer bag coupling part 5, 4 in case the inner bag 3 needs to be changed.

Further, in the embodiments shown in figure 2a and 2b, the outer bag coupling part 4 comprises an inner edge surface 18 disposed around the outer bag main aperture 6, and the inner bag coupling part 5 comprises a further inner edge surface 19 disposed around the inner bag main aperture 7. These inner edge surfaces 19, 18 are arranged to be substantially smooth at their interface once mated for minimizing or even preventing stoma discharge to get caught or trapped in between the inner and outer coupling parts 5,4.

In particular, in an embodiment an inner edge surface 18 of the outer bag coupling part 4 and an inner edge surface 19 of the inner bag coupling part 5 form a continuous surface. This embodiment provides a smooth and continuous surface transition between each of the inner edge surface 18, 19 when the inner and outer bag coupling part 5, 4 are interlocked as shown in figure 2b. As the surface transition does not comprise ridges, ledges or undercuts, stoma discharge will not accumulate on these inner edge surfaces 18, 19 and hygiene and ease of cleaning is improved as a result thereof.

In figure 2b, the interlocking arrangement of the inner and outer bag coupling part 5, 4 is readily achieved by gently pressing the inner bag coupling part 5 onto the outer bag coupling part 4. Doing so may require that the inner and outer bag coupling part 5, 4 are concentrically aligned, allowing the inner and outer bag coupling part 5, 4 to properly mesh as shown.

To facilitate the interlocking of the inner and outer bag coupling part 5, 4 and to increase the ease of use for a user or patient, an embodiment is provided wherein the outer bag coupling part 4 comprises a plurality of outer bag coupling posts 16, 17 and the inner bag coupling part 5 comprises a plurality of alignment apertures 15. The plurality of outer bag coupling posts 16, 17 and the plurality of alignment apertures 15 are configured for aligning the inner bag and outer bag coupling part 5, 4. This embodiment greatly simplifies the interlocking of the inner and outer bag coupling part 5, 4 when a user or patient wishes to replace the inner bag 2. For example, aligning and pushing each outer bag coupling post 16, 17 through a corresponding alignment aperture 15 is straightforward and guarantees that the inner and outer bag coupling part 5, 4 are accurately aligned. Pressing said inner and outer coupling parts 5, 4 into interlocking engagement requires very little effort from the user or patient.

In the embodiments of figure 2a and 2b it is further shown that an outer bag coupling post 16, 17 may comprise a varying cross section, width and/or diameter. For example, in an embodiment the plurality of outer bag coupling posts 16, 17 comprise a first section 16 with a diameter larger than the inner diameter of an inner bag coupling location aperture 15, and a second section 17 with a diameter smaller than the inner diameter of an inner bag coupling location aperture 15. This embodiment ensures a snap and/or snap fit connection between the inner and outer bag coupling part 5, 4, wherein a clicking sound reassures a user or patient that correct interlocking is achieved when pushing an outer bag coupling post 16, 17 through a corresponding alignment aperture 15.

In an alternative embodiment, each of the outer bag coupling posts 16, 17 may comprise a tapered end 16 to facilitate alignment of the inner and outer bag coupling part 5, 4. That is, the tapered end 16 provides a "self-seeking" alignment of an outer bag coupling post 16, 17 with respect to a corresponding alignment aperture 15, so that accurately aligning the inner and outer bag coupling part 5, 4 requires even less effort from a user or patient. In an embodiment, the tapered end 16 of the outer bag coupling post 16, 17 may comprise a spherical or conical profile to facilitate smooth alignment of the inner and outer bag coupling part 5, 4.

In view of the above it is clear that the use of a coupling post 16, 17 and a corresponding alignment aperture 15 can be reversed. More precisely, in an embodiment the inner bag coupling part 5 may comprise a plurality of inner bag coupling posts 16 and the outer bag coupling part 4 may comprise a plurality of alignment apertures 15. In such configuration, the length of the coupling posts 16, 17 should be much smaller, to prevent parts of the ostomy bag assembly sticking into the user's skin. The abovementioned advantages also apply to such an embodiment.

Figure 3 shows an embodiment of a secondary seal 12 according to the present invention. In the embodiment shown, the secondary seal 12 is disposed on the inner bag coupling part 5 around the inner bag main aperture 7. In order to prevent damage to the secondary seal 12 prior to applying a new inner bag 3, the secondary seal 12 may be provided with a release paper 20, which is removable before use. The removable release paper 20 protects the secondary seal 12 from damage, so that optimal adhesion to the outer bag coupling part 4 is ensured. Since the secondary seal 12 also comprises an adhesive tape, the release paper 20 may be disposed on top of the adhesive tape to protect the adhesive surface and prevent accumulation of dirt thereon prior to use of an inner bag 3, e.g. in storage, during transport etc.

In an embodiment, the release paper 20 is provided with a tear off tab at it ends. The tear off tab allows a user or patient to conveniently remove the release paper 20 without touching the adhesive tape 12, which improves hygiene and prevents the adhesive surface from being damaged, smudged etc.

In an advantageous embodiment, as depicted in figure 3, the release paper 20 may comprise two half circular parts. This allows the release paper 20 to be pulled easily as the user or patient need not pull the release paper 20 around the entire circumference of inner bag coupling part 5, which also prevents smudging or damaging the adhesive of the adhesive tape.

Figure 4 shows a cross sectional view of an embodiment of a three position filter device 8 according to the present invention. In the embodiment shown, the three position filter device 8 comprises a shutter member 27 in movable engagement with a filter device body 25 attached to the outer bag 2. The filter device 8 further comprises a filter holder 29 configured to accommodate a gas filter cartridge 21 with a filter 22 to filter out odorous gas components from the inner volume of the ostomy bag assembly.

In an advantageous embodiment, the gas filter cartridge 21 is a replaceable gas filter cartridge, so that the user or patient can maintain optimum filtration capacity of the filtration device 8 and easily dispose a saturated or expired gas filter cartridge 21 with filter 22. Also, the replaceable gas filter cartridge 21 avoids having to replace the entire filter device 8, which reduces replacement intervals for the outer bag 2 and associated costs.

In a further aspect, the present invention may be embodied as a three position filter device 8 only, wherein in a first position the filter device 8 is arranged to guide gas out of the outer bag 2 via a gas filter cartridge 21, in a second position the filter device 8 is arranged to allow unfiltered gas to escape from the outer bag 2, in a third position the filter device 8 is arranged to block gas from escaping from the outer bag 2. The further details of the filter device 8 as described herein can then be considered as further embodiment of such a filter device 8.

In a further embodiment, the filter 22 of the gas filter cartridge 21 may comprise an active gas filtering ingredient, e.g. activated carbon, for a high degree of gas purification. In an even further embodiment, the gas filter cartridge 21 may comprise an active gas filtering ingredient, e.g. active carbon, and a scent ingredient, such as a perfume, a masking scent, and/or fragrances, e.g. lemon, lavender, fruits, forest, pine and vanilla etc. This embodiment further minimizes possible discomfort from particular odorous gas components being discharged from the filter device 8. Again, in an even further aspect, the present invention may be embodied in the gas filter cartridge 21 itself, having the features as described herein.

According to the invention embodiments, the three position filter device 8 provides a first position of the filter device 8 arranged to guide gas out of the outer bag 2 via a gas filter cartridge 21 and filter 22. This would be the most often used position as discharge gases from the stoma outlet are constantly being purified for maximum comfort. A second position of the filter device 8 is provided allowing unfiltered gas to escape from the outer bag 2, thereby making it possible to quickly vent the ostomy bag assembly in case larger volumes of discharge gas would cause "ballooning" of the ostomy bag assembly. The filter device 8 can be completely shut in a third position to block gas from escaping from the outer bag 2. This prevents "pancaking" of the ostomy bag assembly, wherein sides of the outer and/or inner bag 2, 3 ring together as a result of partial vacuum, e.g. being formed when the initial amount of bodily waste sinks to the bottom of the inner bag 3.

The above positions and associated functionalities of the filter device 8 are realized through various openings in the filter device 8 as depicted in figure 4. In particular, in the embodiment shown the filter device 8 comprises a filter passageway extending through a filter intake opening 23, the gas filter cartridge 21 with filter 22, and a filter exhaust opening 30. The filter device 8 further comprises a venting passageway extending through a filter body device opening 26 and a shutter member opening 28.

To utilize the above three positions as well as the filter and venting passageways, the shutter member 27 is in movable engagement with the filter device 8, in particular the filter device body 25. By moving the shutter member 27 with respect to the filter device body 25, one of the three positions as described above can be activated and the filter passageway and/or the venting passageway can be closed or opened separately or simultaneously.

In the embodiment shown in figure 4 the first, second and third positions form a linear arrangement. In this embodiment the shutter member 27 is linearly movable with respect to the filter device body 25, such as linearly sliding the shutter member 27 along the filter device body 25 to change between the three positions. In an alternative embodiment, the first, second and third positions form a circular arrangement. This embodiment may be envisaged wherein the shutter member 27 is rotationally movable with respect to the filter device body 25 to change between the three positions, such as a turning knob to change from one position to another.

Figure 6 shows a side view, partially in cross section, of a further embodiment of the present invention ostomy bag assembly having such a circular arrangement of the first, second and third positions of the filter device 8. Furthermore, figure 7a and 7b show three dimensional views of an upper and lower part of the ostomy bag assembly of Figure 6. In this group of embodiments, the three position device 8 is arranged to have at least three operational positions including a fully open (first position) and fully blocked position (third position), as well as one or more intermediate positions (second position) with a more or less constricted flow. In this group of embodiments, the flow channel from the outer bag 2 to the environment is always through the filter 22.

The embodiment of the ostomy bag assembly as shown in Fig. 6 and 7a-b comprises a filter device 8 with a filter device body 25 designed to be attached to a stoma bag (i.e. outer bag 2, e.g. via a heat seal or ultrasonic weld of a rim or raised energy ring 25b of the filter device body 25). The filter device 8 comprises a filter device body 25 and a mating shutter member 27, wherein the filter device body 25 is provided with a filter intake opening 23 positioned centrally in the filter device body. The shutter member 27 may be provided with suitable ribs 27a or the like, in order to keep the (replaceable) filter 22 in position. The filter 22 in this embodiment comprises a disc of filter material, laminated on both sides with a plastic (gas impermeable) film, having a central aperture 22a (e.g. 2mm diameter) on one side (facing and aligned with the filter intake opening 23 when assembled). As a result, flatus gas entering through the central aperture 22a can exit radially from the center of the filter 22, e.g. in all directions (i.e. 360° degrees) from the central aperture 22a toward an outer circumference of the filter 22.

The filter device body 25 and shutter member 27 are connected together by a connecting element 32. This connecting element 32 (implemented e.g. as a hinge or a strap) prevents possible loss of the shutter member 27, but yet also has sufficient slack to allow a mutual rotational movement of the shutter member 27 with respect to the filter device body 25. The mutual rotational movement may be made easier for a user by the extending lip member 27b as shown in figure 7a. The filter device body 25 and shutter member 27 can be pressed together to form a gas tight assembly. To implement the first, second and third position of the filter device 8, the filter device body 25 is provided with aperture 25a, and the shutter member with (aligned) apertures 28, which are both aligned with the open side surface of filter 22. The apertures may be implemented using a plurality of small ports (as shown in the embodiments of figure 7a-b) or using a slot like configuration (as shown in the embodiment of figure 6).

The ports or slits 25a, 28 in the filter device body 25 and shutter member 27 can be aligned in e.g. three separate positions. Each position of the shutter member 27 relative to the filter device body 25 offers the user a type of flatus gas control. As with the embodiments described above with reference to figures 4 and 5a-c, this embodiment of the ostomy bag assembly can control ballooning and pancaking effects. The insertable filter medium (filter 22) may be compressed inside between the filter device body 25 and shutter member 27. The insertable (e.g. carbon) filter 22 sits in the center of the inner surface of the shutter member 27 and has a free gas passageway around the circumference of the filter 22 which provides a route for the gas that has passed through the (carbon) filter 22 on its way to the ports or slot 25a, 28 which may be open, half open or completely shut.
Position 1 (ports/slot 25a and ports/slot 28 fully aligned) provides a full venting of filtered flatus gas when the patient is comfortable with the rate of flatus produced and its effect on the appliance as a whole.
Position 2 (ports/slot 25a and ports/slot 28 not fully aligned, but in an intermediate position) allows for a degree of control of the rate of gas expulsion.
Position 3 (ports/slot 25a and ports/slot 28 fully misaligned, i.e. not providing any possible passageway) restricts any gas form being expelled and is effective in allowing for some inflation of the bag in a pancaking situation.

Complete removal of the insertable filter 22 (or simply opening the shutter member 27 from the filter device body 25) will allow rapid and discrete venting of the bag, but would e.g. also allow a syringe end to be inserted through into the bag for sluicing or cleaning purposes.

It is noted that the three position filter device 8 of the figure 6 and 7a-b embodiment may be seen as having a first position (filtered output of gas) and a third position (blocking of gas being output from the outer bag) comparable to the same positions as described with respect to the figure 4 and 5a-c embodiment, as well as a second position allowing unfiltered gas to escape from the outer bag 2 (by removing the shutter member 27 from the filter device body 25). In addition, this group of embodiments can then be seen as having a further (plurality of) intermediate positions where a constricted flow of gas is allowed to pass from the outer bag 2 through the filter 22.

The various parts of the filter device 8 may be easily made e.g. using injection moulding techniques. Further elements may be provided for easy assembly of the filter device, such as a frame 31 connecting the filter device body 25 and filter holder 29. Also, optionally, a liner element 24 may be provided in the area around filter intake opening 23 which is of a skin friendly material.

Figure 5a to 5c show embodiments for each position of the three position filter device according to the present invention.

In particular, figure 5a shows an embodiment of the three position filter device 8 in the first position according to the present invention. In the embodiment shown, the first position of the shutter member 27 corresponds to a midway point with respect to its sliding freedom. That is, in the first position the shutter member 8 is able to move to the left and to the right. In the first position as shown in figure 5a, the shutter member 27 is positioned over the filter body device opening 26 and as such blocks any gas discharge there through. Further, the shutter member opening 28 is positioned over the filter device body 25 and as such is blocked. On the other hand, the filter exhaust opening 30 remains open and allows discharge gases to pass through the filter device 8 as depicted.

Figure 5b shows an embodiment of the three position filter device in the second position according to the present invention. In the embodiment shown, the shutter element 27 is positioned to the left of its midway point. In this position the shutter member opening 28 is aligned with the filter device body opening 26. As a result the venting passageway is opened and discharge gases in the outer bag 2 can be now be vented immediately without passing through the gas filter cartridge 21. The second position is particularly advantageous for preventing ballooning of the ostomy bag assembly, thus wherein an excess volume of discharge gases is produced exceeding a maximum flow capacity of the gas filter cartridge 21. Note that in the second position the filter intake opening 23 and filter exhaust opening 30 remain open, so gases are able to pass through both the venting passageway as well as the filter passageway.

Figure 5c shows an embodiment of the three position filter device in third position according to the present invention. In the embodiment shown, the shutter element 27 is positioned to the right of its midway point. In this position the shutter member opening 28 is positioned over the filter device body opening 26 as well as the filter exhaust opening 30, thus blocking discharge of gases. Also, the shutter member opening 28 is positioned over the filter holder 29 and as such is being blocked thereby. I the third position as depicted in figure 5c, both the filter passageway and the venting passageway are blocked. This embodiment is particularly advantageous to prevent "pancaking" of the ostomy bag assembly, wherein the development of a vacuum within the ostomy bag assembly can be prevented that would otherwise ring sides of the inner and/or outer bag 2, 3 together.

In an alternative embodiment the shutter member 27 may well be implemented as being in rotational engagement with the filter device body 25, wherein turning the shutter element 27 allows different positions to be selected to open or close the filter passageway and/or the venting passageway.

The present invention embodiments have been described above with reference to a number of exemplary embodiments as shown in the drawings. Modifications and alternative implementations of some parts or elements are possible, and are included in the scope of protection as defined in the appended claims.

## Claims

1. Ostomy bag assembly comprising
an outer bag (2) with a base plate coupling element (1) attached to an edge of an outer bag main aperture (6),
an inner bag (3) with an inner bag main aperture (7), which in operation is aligned with the outer bag main aperture (6), the inner bag (3) being provided with a liquid sealing and gas permeable barrier device (9) designed to allow gas to escape the inner bag to inflate the outer bag so as to prevent pancaking of the outer bag,
wherein the outer bag (2) is provided with a three position filter device (8), wherein the three position filter device (8) is arranged to have a first, second and third operational position each allowing a different amount of gas to be guided out from the outer bag (2),
wherein the three position filter device (8) comprises a shutter member (27) in movable engagement with a filter body (25) attached to the outer bag (2), and
wherein the first, second and third positions form a linear or circular arrangement and the shutter member (27) being linearly or rotationally moveable with respect to the filter body (25), respectively, so as to select between said first, second and third positions.

2. Ostomy bag assembly according to claim 1, wherein in a first position the filter device (8) is arranged to guide gas out of the outer bag (2) via a gas filter cartridge (21), in a second position the filter device (8) is arranged to allow unfiltered gas to escape from the outer bag (2), and in a third position the filter device (8) is arranged to block gas from escaping from the outer bag (2).

3. Ostomy bag assembly according to claim 1, wherein in a first position the filter device (8) is arranged to guide gas out of the outer bag (2) via a gas filter cartridge (21), in a second position the filter device (8) is arranged to guide gas out of the outer bag (2) via a gas filter cartridge (21) with a constricted flow, and in a third position the filter device (8) is arranged to block gas from escaping from the outer bag (2).

4. Ostomy bag assembly according to any one of claim 1-3, wherein the gas filter cartridge (21) is a replaceable gas filter cartridge.

5. Ostomy bag assembly according to any one of claims 1-4, wherein the gas filter cartridge (21) comprises an active gas filtering ingredient and optionally a scent ingredient.

6. Ostomy bag assembly according to any one of claims 1-5, wherein the filter device (8) is positioned on a top part of the outer bag (2).

7. Ostomy bag assembly according to any one of claims 1-6, wherein the filter device (8) is positioned on the outer bag (2) on the same side as the outer bag main aperture (6).

8. Ostomy bag assembly according to any one of claims 1-7, wherein a coupling between the inner bag (3) and the outer bag (3) is provided by a sealing arrangement,
the inner bag (3) comprising an inner bag coupling part (14) surrounding the inner bag main aperture (7), and
the outer bag (2) comprising an outer bag coupling part (13) surrounding the outer bag main aperture (6), the sealing arrangement
comprising an O-ring seal (11) and a secondary seal (12), wherein the secondary seal (12) comprises an adhesive tape attached to respective mating surfaces on the inner bag coupling part (14) and the outer bag coupling part (13).

9. Ostomy bag assembly according to claim 8, wherein the adhesive tape is of an easy-to-remove type.

10. Ostomy bag assembly according to claim 8 or 9, wherein the secondary seal (12) is provided with a release paper (20), which is removable directly before use.

11. Ostomy bag assembly according to any one of claims 8-10, wherein an inner edge surface (18) of the outer bag coupling part (13) and an inner edge surface (19) of the inner bag coupling part (14) form a continuous surface.

12. Ostomy bag assembly according to any one of claims 8-11, wherein the outer bag coupling part (13) comprises a plurality of outer bag coupling posts (16, 17) and the inner bag coupling part (14) comprises a plurality of alignment apertures (15), wherein
the plurality of outer bag coupling posts (16, 17) and the plurality of alignment apertures (15) are configured for aligning the inner bag coupling part (14) and outer bag coupling part (13).

13. Ostomy bag assembly according to claim 12, wherein the one or more outer bag coupling posts (16, 17), having a first section (16) with a diameter larger than the inner diameter of an inner bag coupling location aperture (15) and a second section (17) with a diameter smaller than the inner diameter of an inner bag coupling location aperture (15).

## Patentansprüche

1. Ostomiebeutelanordnung, umfassend
einen äußeren Beutel (2) mit einem an einem Rand einer Hauptöffnung (6) des äußeren Beutels angebrachten Basisplattenverbindungselement (1),
einen inneren Beutel (3) mit einer Hauptöffnung (7) des inneren Beutels, die im Einsatz an der Hauptöffnung (6) des äußeren Beutels axial ausgerichtet ist, wobei der innere Beutel (3) mit einer flüssigkeitsabdichtenden und gasdurchlässigen Barrierevorrichtung (9) versehen ist, die so ausgestaltet ist, dass sie Gas aus dem inneren Beutel austreten lässt, um den äußeren Beutel aufzublasen, um ein Zusammensacken des äußeren Beutels zu verhindern,
wobei der äußere Beutel (2) mit einer Dreipositionsfiltervorrichtung (8) versehen ist, wobei die Dreipositionsfiltervorrichtung (8) so angeordnet ist, dass sie eine erste, zweite und dritte Einsatzposition aufweist, von denen jede das Herausführen einer unterschiedlichen Menge an Gas aus dem äußeren Beutel (2) ermöglicht,
wobei die Dreipositionsfiltervorrichtung (8) ein Verschlussglied (27) in bewegbarem Eingriff mit einem an dem äußeren Beutel (2) angebrachten Filterkörper (25) umfasst und
wobei die erste, zweite und dritte Position eine lineare oder kreisförmige Anordnung bilden und das Verschlussglied (27) schiebbar beziehungsweise drehbar in Bezug auf den Filterkörper (25) bewegbar ist, um zwischen der ersten, zweiten und dritten Position zu wählen.

2. Ostomiebeutelanordnung nach Anspruch 1, wobei in einer ersten Position die Filtervorrichtung (8) so angeordnet ist, dass sie Gas über eine Gasfilterpatrone (21) aus dem äußeren Beutel (2) herausführt, in einer zweiten Position die Filtervorrichtung (8) so angeordnet ist, dass sie den Austritt von ungefiltertem Gas aus dem äußeren Beutel (2) ermöglicht, und in einer dritten Position die Filtervorrichtung (8) so angeordnet ist, dass sie den Austritt von Gas aus dem äußeren Beutel (2) blockiert.

3. Ostomiebeutelvorrichtung nach Anspruch 1, wobei in einer ersten Position die Filtervorrichtung (8) so angeordnet ist, dass sie Gas über eine Gasfilterpatrone (21) aus dem äußeren Beutel (2) herausführt, in einer zweiten Position die Filtervorrichtung (8) so angeordnet ist, dass sie Gas über eine Gasfilterpatrone (21) mit einem verengten Strom aus dem äußeren Beutel (2) herausführt, und in einer dritten Position die Filtervorrichtung (8) so angeordnet ist, dass sie den Austritt von Gas aus dem äußeren Beutel (2) blockiert.

4. Ostomiebeutelanordnung nach einem der Ansprüche 1 bis 3, wobei die Gasfilterpatrone (21) eine austauschbare Gasfilterpatrone ist.

5. Ostomiebeutelanordnung nach einem der Ansprüche 1 bis 4, wobei die Gasfilterpatrone (21) einen aktiven Gasfilterbestandteil und optional einen Duftbestandteil umfasst.

6. Ostomiebeutelanordnung nach einem der Ansprüche 1 bis 5, wobei die Filtervorrichtung (8) an einem oberen Teil des äußeren Beutels (2) positioniert ist.

7. Ostomiebeutelanordnung nach einem der Ansprüche 1 bis 6, wobei die Filtervorrichtung (8) an dem äußeren Beutel (2) auf derselben Seite positioniert ist wie die Hauptöffnung (6) des äußeren Beutels.

8. Ostomiebeutelanordnung nach einem der Ansprüche 1 bis 7, wobei durch eine Dichtungsanordnung eine Verbindung zwischen dem inneren Beutel (3) und dem äußeren Beutel (3) bereitgestellt wird,
wobei der innere Beutel (3) einen die Hauptöffnung (7) des inneren Beutels umgebenden Verbindungsteil (14) des inneren Beutels umfasst und
der äußere Beutel (2) einen die Hauptöffnung (6) des äußeren Beutels umgebenden Verbindungsteil (13) des äußeren Beutels umfasst,
wobei die Dichtungsanordnung eine O-Ring-Dichtung (11) und eine Sekundärdichtung (12) umfasst, wobei die Sekundärdichtung (12) ein Klebeband umfasst, das an jeweils zusammenpassenden Oberflächen des Verbindungsteils (14) des inneren Beutels und des Verbindungsteils (13) des äußeren Beutels angebracht ist.

9. Ostomiebeutelanordnung nach Anspruch 8, wobei das Klebeband ein leicht zu entfernendes Klebeband ist.

10. Ostomiebeutelanordnung nach Anspruch 8 oder 9, wobei die Sekundärdichtung (12) mit einem Trennpapier (20) versehen ist, das unmittelbar vor der Benutzung entfernbar ist.

11. Ostomiebeutelanordnung nach einem der Ansprüche 8 bis 10, wobei eine Innenrandoberfläche (18) des Verbindungsteils (13) des äußeren Beutels und eine Innenrandoberfläche (19) des Verbindungsteils (14) des inneren Beutels eine durchgehende Oberfläche bilden.

12. Ostomiebeutelanordnung nach einem der Ansprüche 8 bis 11, wobei der Verbindungsteil (13) des äußeren Beutels eine Mehrzahl von Verbindungsstäben (16, 17) des äußeren Beutels und der Verbindungsteil (14) des inneren Beutels eine Mehrzahl von Ausrichtungsöffnungen (15) umfasst, wobei
die Mehrzahl von Verbindungsstäben (16, 17) des äußeren Beutels und die Mehrzahl von Ausrichtungsöffnungen (15) ausgeführt sind zum Ausrichten des Verbindungsteils (14) des inneren Beutels und Verbindungsteils (13) des äußeren Beutels aneinander.

13. Ostomiebeutelanordnung nach Anspruch 12, wobei der eine oder die mehreren Verbindungsstäbe (16, 17) des äußeren Beutels einen ersten Abschnitt (16) mit einem Durchmesser größer als der Innendurchmesser einer Verbindungsstellenöffnung (15) des inneren Beutels und einen zweiten Abschnitt (17) mit einem Durchmesser kleiner als der Innendurchmesser einer Verbindungsstellenöffnung (15) des inneren Beutels aufweisen.

## Revendications

1. Ensemble formant poche de stomie comprenant
une poche externe (2) pourvue d'un élément de couplage de plaque de base (1) fixé à un bord d'une ouverture principale de poche externe (6),
une poche interne (3) pourvue d'une ouverture principale de poche interne (7), qui en fonctionnement est alignée avec l'ouverture principale de poche externe (6), la poche interne (3) étant pourvue d'un dispositif de barrière étanche aux liquides et perméable au gaz (9) conçu pour permettre au gaz de s'échapper de la poche interne pour gonfler la poche externe afin d'empêcher l'aplatissement de la poche externe,
dans lequel la poche externe (2) est pourvue d'un dispositif de filtrage à trois positions (8), dans lequel le dispositif de filtrage à trois positions (8) est agencé de sorte à avoir une première, une deuxième et une troisième position opérationnelle, chacune permettant à une quantité différente de gaz d'être guidée hors de la poche externe (2),
dans lequel le dispositif de filtrage à trois positions (8) comprend un élément d'obturation (27) en prise mobile avec un corps de filtre (25) fixé à la poche externe (2), et
dans lequel les première, deuxième et troisième positions forment un agencement linéaire ou circulaire et l'élément d'obturation (27) est mobile de manière coulissante et rotationnelle par rapport au corps de filtre (25), de manière respective, de sorte à choisir entre lesdites première, deuxième et troisième positions.

2. Ensemble formant poche de stomie selon la revendication 1, dans lequel dans une première position, le dispositif de filtrage (8) est agencé pour guider le gaz hors de la poche externe (2) via une cartouche de filtre à gaz (21), dans une deuxième position, le dispositif de filtrage (8) est agencé de manière à permettre au gaz non filtré de s'échapper de la poche externe (2), et dans une troisième position, le dispositif de filtrage (8) est agencé de manière à bloquer le gaz pour l'empêcher de s'échapper de la poche externe (2).

3. Ensemble formant poche de stomie selon la revendication 1, dans lequel dans une première position, le dispositif de filtrage (8) est agencé pour guider le gaz hors de la poche externe (2) via une cartouche de filtre à gaz (21), dans une deuxième position, le dispositif de filtrage (8) est agencé pour guider le gaz hors de la poche externe (2) via une cartouche de filtre à gaz (21) avec un écoulement limité, et dans une troisième position, le dispositif de filtrage (8) est agencé pour bloquer le gaz afin de l'empêcher de s'échapper de la poche externe (2).

4. Ensemble formant poche de stomie selon l'une quelconque des revendications 1 à 3, dans lequel la cartouche de filtre à gaz (21) est une cartouche de filtre à gaz remplaçable.

5. Ensemble formant poche de stomie selon l'une quelconque des revendications 1 à 4, dans lequel la cartouche de filtre à gaz (21) comprend un ingrédient actif de filtrage des gaz et éventuellement un ingrédient parfumé.

6. Ensemble formant poche de stomie selon l'une quelconque des revendications 1 à 5, dans lequel le dispositif de filtrage (8) est positionné sur une partie supérieure de la poche externe (2).

7. Ensemble formant poche de stomie selon l'une quelconque des revendications 1 à 6, dans lequel le dispositif de filtrage (8) est positionné sur la poche externe (2) du même côté que l'ouverture principale de poche externe (6).

8. Ensemble formant poche de stomie selon l'une quelconque des revendications 1 à 7, dans lequel un couplage entre la poche interne (3) et la poche externe (3) est assuré par un agencement d'étanchéité,
la poche interne (3) comprenant une partie de couplage de poche interne (14) entourant l'ouverture principale de la poche interne (7), et
la poche externe (2) comprenant une partie de couplage de poche externe (13) entourant l'ouverture principale de la poche externe (6),
l'agencement d'étanchéité comprenant un joint torique (11) et un joint secondaire (12), dans lequel le joint secondaire (12) comprend un ruban adhésif fixé aux surfaces de contact respectives sur la partie de couplage de la poche interne (14) et la partie de couplage de la poche externe (13).

9. Ensemble formant poche de stomie selon la revendication 8, dans lequel le ruban adhésif est d'un type facile à retirer.

10. Ensemble formant poche de stomie selon la revendication 8 ou 9, dans lequel le joint secondaire (12) est pourvu d'un papier antiadhésif (20), qui peut être enlevé directement avant usage.

11. Ensemble formant poche de stomie selon l'une quelconque des revendications 8 à 10, dans lequel une surface de bord interne (18) de la partie de couplage de la poche externe (13) et une surface de bord interne (19) de la partie de couplage de la poche interne (14) forment une surface continue.

12. Ensemble formant poche de stomie selon l'une quelconque des revendications 8 à 11, dans lequel la partie de couplage de la poche externe (13) comprend une pluralité de montants de couplage de poche externe (16, 17) et la partie de couplage de la poche interne (14) comprend une pluralité d'ouvertures d'alignement (15), dans lequel
la pluralité de montants de couplage de poche externe (16, 17) et la pluralité d'ouvertures d'alignement (15) sont configurés pour aligner la partie de couplage de poche interne (14) et la partie de couplage de poche externe (13).

13. Ensemble formant poche de stomie selon la revendication 12, dans lequel les un ou plusieurs montants de couplage de poche externe (16, 17), ayant une première section (16) d'un diamètre supérieur au diamètre interne d'une ouverture d'emplacement de couplage de la poche interne (15) et une seconde section (17) d'un diamètre inférieur au diamètre interne d'une ouverture d'emplacement de couplage de poche interne (15).
